# EUROPEAN PATENT APPLICATION

(11) **EP 4 613 293 A1**
(43) Date of publication of application: **10.09.2025**
(21) Application number: 23886401.1
(22) Date of filing: 03.11.2023
(51) Int. Cl.: A61K 48/00, A61K 31/7088, A61K 35/28, A61P 9/00, C12N 5/0775

(54) **COMPOSITION FOR PREVENTING OR TREATING MOYAMOYA DISEASE**

(30) Priority: 03.11.2022 KR 20220145550
(71) Applicant: S&E Bio Co., Ltd., Seoul 06351 (KR)
(72) Inventor: KIM, Eun Hee, Seoul 06351 (KR); LEE, Woo Joo, Seoul 06351 (KR); OH, Gyun Sik, Seoul 06351 (KR); LEE, Na Eun, Seoul 06351 (KR)
(74) Representative: Cabinet Becker et Associés
(86) International application number: PCT/KR2023/017597
(87) International publication number: WO 2024/096707

(57) **Abstract**

The present invention relates to: a composition for preventing or treating Moyamoya disease, comprising, as an active ingredient, miRNAs or extracellular vesicles comprising same; and a composition for preventing or treating Moyamoya disease, comprising extracellular vesicles derived from a three-dimensional spheroid-type cell aggregate prepared by means of a novel preparation method. If miRNAs or extracellular vesicles comprising same, of the present invention, are used, the expression of miRNAs, which are downregulated in patients with Moyamoya disease or Moyamoya syndrome, is increased such that vascular anastomoses, an increase in cerebral blood vessel diameter, and an increase in cerebral blood flow can be induced. Therefore, miRNAs or extracellular vesicles comprising same, of the present invention, can be used as a novel therapeutic agent for treating Moyamoya disease or Moyamoya syndrome.

## Description

### [Technical Field]

The present disclosure relates to a composition for preventing or treating Moyamoya disease, including miRNAs; or extracellular vesicles including the same as an active ingredient, and a composition for preventing or treating Moyamoya disease, including extracellular vesicles derived from a three-dimensional spheroid-type cell aggregate prepared by a novel preparation method.

### [Background Art]

Treatments using stem cells, particularly mesenchymal stem cells (MSCs), and positive clinical results have been reported for various diseases. However, stem cell therapy has a risk of cell-related side effects such as vascular occlusion, tumor formation, and coagulation disorders, and efficacy verification through clinical trials is still required. It is known that a paracrine effect of stem cells induces the promotion of the regeneration and vascular regeneration of surrounding skin cells, and in particular, extracellular vesicles (EVs) are known as a major effector in the paracrine effect.

The extracellular vesicles are classified into exosomes and microvesicles depending on a size, and the exosomes have the diameter of 30 to 150 nm, and the microvesicles have the size of 100 to 1,000 nm. The extracellular vesicles are parts of the cell membrane that have been separated into the blood, and are known to mediate intercellular communication by including both protein and nuclear components. Using extracellular vesicles instead of stem cells not only increase safety by minimizing side effects caused by using stem cells, but also is advantageous in terms of biodistribution and production process.

However, mass production, obtaining method, and the like for using extracellular vesicles derived from stem cells have not yet been established, and much research has been not conducted on methods for further enhancing the efficacy of extracellular vesicles while maintaining the characteristics of stem cell-derived extracellular vesicles.

Moyamoya disease is a specific cerebrovascular disease in which the arteries supplying blood to the brain gradually narrow and shrink, reducing the amount of blood going to the brain, which is also called internal carotid artery hypoplasia. Moyamoya disease is a cerebrovascular disease of unknown cause in which stenosis and occlusion occur in the terminal part of a specific blood vessel in the brain, that is, the internal carotid artery, without any specific cause, and the thick blood vessels narrow to form a network of thin blood vessels. The disease was named Moyamoya disease, which means "puff of smoke" in Japanese, because the thin blood vessels appear like smoke or clouds. Moyamoya disease is a disease that occurs frequently in Japan, Korea, and China, and particularly, is known to occur frequently in Korea and Japan.

The overall exact pathogenesis or cause of Moyamoya disease has not been identified, but recently, a c.14429G>A (p.Arg4810Lys) variant of an RNF213 gene has been identified as an important genetic factor. In Japanese patients with Moyamoya disease, the variant was found in about 95% of familial patients and about 73% of sporadic patients, and the variant was found at a very low frequency of about 1.4% in a normal group. The variant was also identified as an important genetic cause of Moyamoya disease in Korean patients with Moyamoya disease.

For the treatment of Moyamoya disease, the most fundamental treatment for Moyamoya disease is to dilate the narrowed blood vessels again, thereby increasing the blood flow to the brain. However, no drug has yet been developed as a therapeutic agent that has the effect, and treatment is performed only by surgical treatment.

For the treatment of Moyamoya disease, a surgical treatment method has been representatively conducted to improve blood flow within the cranial cavity through anastomosis of attempting to connect normal blood vessels outside the cranial cavity with abnormal blood vessels inside the cranial cavity. Depending on a surgical method, the vascular anastomosis is divided into direct vascular anastomosis that directly connects the intracranial and extracranial blood vessels, and indirect vascular anastomosis that does not directly connect the intracranial and extracranial blood vessels. The vascular anastomosis is a direct surgery of dissecting the extracranial blood vessels, removing the skull, and finding and connecting the abnormal intracranial blood vessels, and an indirect surgery in which the process is the same, but the intracranial and extracranial blood vessels are not directly connected. Therefore, general anesthesia was necessary, the surgical time was long, and the surgeon's skilled techniques were required. In particular, the direct anastomosis is highly dependent on the surgeon, and even in trustworthy medical institutions, there were cases where the postoperative cerebral infarction rate reached about 21% and the mortality rate reached 9%. Indirect vascular anastomosis also had the advantage of being relatively simple compared to direct anastomosis, but had the disadvantage of being applicable only to pediatric patients, as the environment and conditions for cerebral angiogenesis are known to be superior to those of adults, and being not applied to adult patients. In addition, although the indirect vascular anastomosis is a relatively simple surgery compared to direct vascular anastomosis, surgical side effects still occur, and about 15% of patients may have problems such as cerebral infarction after surgery. Therefore, it was difficult to even attempt treatment for acute stroke.

Therefore, there is a need for novel non-surgical treatment methods and therapeutic agents that may effectively treat Moyamoya disease through non-surgical methods.

### [Disclosure]

### [Technical Problem]

Accordingly, the present inventors identified that the expression of at least one miRNA selected from the group consisting of miR-19b-3p, miR-99a-5p, miR-100-5p, miR-101-3p, and miR-122-5p was specifically reduced in patients with Moyamoya disease while studying the treatment of Moyamoya disease, and identified that at least one miRNA selected from the group consisting of miR-19b-3p, miR-99a-5p, miR-100-5p, miR-101-3p, and miR-122-5p or extracellular vesicles abundantly including miRNA may be used as novel therapeutic agents for the treatment of Moyamoya disease. In particular, the present inventors confirmed that extracellular vesicles derived from a three-dimensional spheroid-type cell aggregate (3D-static-spheroid) newly designed by the present inventors included a large amount of miRNA, and that treatment with the extracellular vesicles may effectively treat Moyamoya disease, and then completed the present disclosure.

Therefore, an object of the present disclosure is to provide a composition for preventing or treating Moyamoya disease or Moyamoya syndrome, including, as an active ingredient, at least one miRNA selected from the group consisting of miR-19b-3p, miR-99a-5p, miR-100-5p, miR-101-3p and miR-122-5p; or extracellular vesicles including the miRNA.

### [Technical Solution]

In order to achieve the object, an aspect of the present disclosure provides a pharmaceutical composition for use in preventing or treating Moyamoya disease or Moyamoya syndrome, including extracellular vesicles including at least one miRNA selected from the group consisting of miR-19b-3p, miR-99a-5p, miR-100-5p, miR-101-3p and miR-122-5p.

In addition, another aspect of the present disclosure provides a pharmaceutical composition for use in preventing or treating Moyamoya disease or Moyamoya syndrome, including extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

In addition, yet another aspect of the present disclosure provides a pharmaceutical composition for use in preventing or treating Moyamoya disease or Moyamoya syndrome, including at least one miRNA selected from the group consisting of miR-19b-3p, miR-99a-5p, miR-100-5p, miR-101-3p and miR-122-5p.

In addition, still another aspect of the present disclosure provides an *in vitro* composition for use in promoting vascular anastomosis of vascular endothelial cells induced by mutation or deletion of a Ring finger protein 213 (RNF213) gene, including extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

In addition, still another aspect of the present disclosure provides a method for preparing a composition for preventing or treating Moyamoya disease or Moyamoya syndrome including extracellular vesicles derived from a three-dimensional spheroid-type cell aggregate, including (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

In addition, still another aspect of the present disclosure provides a method for preventing or treating Moyamoya disease or Moyamoya syndrome, including administering to a subject in need thereof extracellular vesicles including at least one miRNA selected from the group consisting of miR-19b-3p, miR-99a-5p, miR-100-5p, miR-101-3p and miR-122-5p.

In addition, still another aspect of the present disclosure provides a method for preventing or treating Moyamoya disease or Moyamoya syndrome, including administering to a subject in need thereof extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

### [Advantageous Effects]

According to the present disclosure, the expression of miRNAs, which are down-regulated in patients with Moyamoya disease or Moyamoya syndrome, is increased by using the miRNAs or extracellular vesicles including the miRNAs, thereby inducing vascular anastomoses, an increase in cerebral blood vessel diameter, and an increase in cerebral blood flow. Therefore, the miRNAs or the extracellular vesicles including the miRNAs of the present disclosure may be used as a novel therapeutic agent for treating Moyamoya disease or Moyamoya syndrome.

### [Description of Drawings]

FIG. 1 is a schematic diagram illustrating a process of preparing a 3D spheroid-type cell aggregate and isolating extracellular vesicles therefrom.
FIG. 2A is a diagram of observing a microwell including a 3D-dynamic-PEG spheroid culture medium.
FIG. 2B is a diagram of observing a microwell including a 3D-static-spheroid culture medium.
FIG. 2C is a diagram illustrating changes in aggregate area according to culture periods of a 3D-dynamic-PEG spheroid and a 3D-static-spheroid prepared using a microwell.
FIG. 3 is a diagram of comparing size distributions of a 3D-static-spheroid and a 3D-dynamic-PEG spheroid.
FIG. 4 is a diagram of observing the shape of 3D-static-spheroid EV with an electron microscope.
FIG. 5 is a diagram illustrating results of nanoparticle tracking analysis (NTA) to confirm the concentration and size distribution of 3D-static-spheroid EV.
FIG. 6 is a diagram illustrating results of ELISA and Western blot to confirm expression markers of 3D-static-spheroid EV.
FIG. 7 is a diagram of comparing productions of 3D-static-spheroid EV, 3D-dynamic-PEG spheroid EV, and 2D-EV per derived cell.
FIG. 8 is a diagram illustrating a result of confirming the size, roundness, and solidity of 3D spheroids prepared after varying the diameter and depth of a microwell and the number of cells per well in the preparation of 3D-static spheroid EV.
FIG. 9 is a diagram illustrating a result of comparing the expression levels of miRNA 132 and miRNA 210 expressed in 3D-static-spheroid EV and 2D-EV prepared under various conditions.
FIG. 10 illustrates results of confirming the expression of miR-101-3p and miR-19b-3p in a normal control, intracranial arteriosclerosis (ICAS), and Moyamoya disease (MMD).
FIG. 11 illustrates results of confirming the expression of miR-100-5p, miR-122-5p, and miR-99a-5p in a normal control and Moyamoya disease (MMD) through small RNA sequencing.
FIG. 12 illustrates results of confirming the expression of miR-100-5p, miR-122-5p, and miR-99a-5p in a normal control and Moyamoya disease (MMD) through quantitative PCR (*p < 0.05, **p < 0.01).
FIG. 13 is a diagram illustrating results of confirming the expression of miR-101-3p and miR-19b-3p in 2D-EV and 3D-static-spheroid EV (3D-EV).
FIG. 14 is a diagram illustrating results of confirming the expression of miR-122-5p in 2D-EV and 3D-static-spheroid EV (3D-EV).
FIG. 15 is a diagram illustrating results of confirming the angiogenic ability according to treatment with 3D-static-spheroid EV (indicated as EV) in a Moyamoya disease model fabricated through RNF213 siRNA treatment.
FIG. 16 is a diagram illustrating results of confirming the vascular endothelial cell proliferation ability according to treatment with 3D-static-spheroid EV (indicated as EV) in a Moyamoya disease model fabricated through RNF213 siRNA treatment.
FIG. 17 is a diagram illustrating results of confirming changes in the expression of miR-101-3p and miR-19b-3p in vascular endothelial cells according to treatment with 3D-static-spheroid EV (indicated as EV) in a Moyamoya disease model manufactured through RNF213 siRNA treatment.
FIG. 18 is a schematic diagram illustrating an experimental protocol using a Moyamoya disease-mimetic model.
FIG. 19A is an image of cerebral blood flow changes using laser speckle imaging (3D-EV: 3D-static-spheroid EV administration group).
FIG. 19B is a diagram illustrating results of quantifying cerebral blood flow changes according to 3D-static-spheroid EV administration (BCAS EV) by a regions of interest (ROI) average value (*p < 0.05).
FIG. 20 is a diagram illustrating results of confirming changes in cerebral blood vessels according to PBS administration and 3D-static-spheroid EV administration group (BCAS EV) in a Moyamoya disease-mimetic model using cerebral blood vessel staining.
FIG. 21A is a diagram illustrating results of confirming changes in diameter of the Circle of Willis (CoW) according to PBS administration and 3D-static-spheroid EV administration groups (EV) in a Moyamoya disease-mimetic model (*p < 0.05, **p < 0.01).
FIG. 21B is a diagram illustrating the number of vascular anastomoses according to PBS administration and 3D-static-spheroid EV administration groups (EV) in a Moyamoya disease-mimetic model (**p < 0.01).

### [Best Mode]

The present disclosure relates to a pharmaceutical composition for preventing or treating Moyamoya disease or Moyamoya syndrome, including extracellular vesicles including at least one miRNA selected from the group consisting of miR-19b-3p, miR-99a-5p, miR-100-5p, miR-101-3p, and miR-122-5p, or a method for preventing or treating Moyamoya disease or Moyamoya syndrome using the same.

Further, the present disclosure relates to a pharmaceutical composition for preventing or treating Moyamoya disease or Moyamoya syndrome and a method for treating Moyamoya disease or Moyamoya syndrome using the same, including extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

Hereinafter, the present disclosure will be described in detail.

The present disclosure provides a composition for preventing, alleviating or treating Moyamoya disease or Moyamoya syndrome, including extracellular vesicles including at least one miRNA selected from the group consisting of miR-19b-3p, miR-99a-5p, miR-100-5p, miR-101-3p and miR-122-5p; or a pharmaceutical composition for preventing or treating Moyamoya disease or Moyamoya syndrome, including extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

When prepared by the preparation method, the extracellular vesicles including at least one miRNA selected from the group consisting of miR-19b-3p, miR-99a-5p, miR-100-5p, miR-101-3p, and miR-122-5p of the present disclosure may be used in the same meaning as extracellular vesicles derived from a three-dimensional spheroid-type cell aggregate, and in this case, may be referred to as 3D-static-spheroid EVs.

In addition, in comparison, the extracellular vesicles derived from the 3D dynamically cultured spheroid-type cell aggregate may be used interchangeably with "3D-dynamic-PEG-spheroid-EVs".

In the present disclosure, the "extracellular vesicles derived from 2D-cultured mesenchymal stem cells" may include any extracellular vesicle obtained by culturing stem cells according to a conventional 2D culture method and isolated by a conventional method of isolating extracellular vesicles without limitation. In an example of the present disclosure, the conventional 2D culture method was performed by a method of washing stem cells cultured for 3 days in a cell stack with PBS, replacing the medium with a serum-free medium, and further culturing the cells for 2 days.

The three-dimensional spheroid-type cell aggregate of the present disclosure may have higher kurtosis in size distribution than the "spheroid-type cell aggregate obtained by 3D dynamic-culturing the mesenchymal stem cells". Therefore, the size of the three-dimensional spheroid-type cell aggregate is smaller than that of the "spheroid-type cell aggregate obtained by 3D dynamic-culturing the mesenchymal stem cells" and may have a relatively uniform size distribution.

As used in the present disclosure, the 'extracellular vesicles of the present disclosure' may include both 'extracellular vesicles including at least one miRNA selected from the group consisting of miR-19b-3p, miR-99a-5p, miR-100-5p, miR-101-3p and miR-122-5p' and 'extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate'.

The Accession numbers and sequence information of miR-19b-3p, miR-99a-5p, miR-100-5p, miR-101-3p and miR-122-5p of the present disclosure are shown in Table 4.

As used in the present disclosure, the miRNA refers to untranslated RNA of approximately 22 nt that acts as a post-transcriptional repressor through base binding to a 3' untranslated region (UTR) of mRNA.

The miR-19b-3p, miR-99a-5p, miR-100-5p, miR-101-3p and miR-122-5p are characterized as miRNAs that are significantly down-expressed in extracellular vesicles, such as exosomes and microvesicles, derived from plasma of Moyamoya patients compared to a normal group, and when these miRNAs are administered as a therapeutic agent to a subject in need of treatment for Moyamoya disease or Moyamoya syndrome, the expression levels of the miRNAs increase, thereby achieving a therapeutic effect for Moyamoya disease or syndrome.

The extracellular vesicles of the present disclosure may be characterized by highly expressing the at least one miRNA compared to naturally secreted extracellular vesicles or 2D cultured stem cell-derived extracellular vesicles.

Here, the naturally secreted extracellular vesicles refer to extracellular vesicles naturally secreted by cells without separate treatment, and the 2D cultured stem cell-derived extracellular vesicles may be extracellular vesicles prepared by 2D culture methods commonly known in the art without limitation, for example, a method of Example 7.

The extracellular vesicles of the present disclosure may preferably be extracellular vesicles derived from a three-dimensional spheroid-type cell aggregate prepared by a method including the following steps: (a) preparing a three-dimensional spheroid-type cell aggregate by 3-dimensionally (3D) static culturing stem cells in a microwell with a diameter of 200 to 800 µm and a depth of 100 to 1,000 µm; and (b) isolating extracellular vesicles from the three-dimensional spheroid-type cell aggregate.

The extracellular vesicles obtained from the three-dimensional spheroid-type cell aggregate may exhibit different miRNA expression patterns and characteristics from naturally secreted extracellular vesicles or 2D cultured stem cell-derived extracellular vesicles, and particularly, include abundant miRNAs that are down-expressed in patients with Moyamoya disease or Moyamoya syndrome. Due to these characteristics, when the extracellular vesicles of the present disclosure are administered to a subject in need thereof, the down-expressed miRNAs may be increased, thereby effectively achieving a therapeutic effect of Moyamoya disease or syndrome.

The miRNA as the active ingredient included in the extracellular vesicles of the present disclosure may be effectively delivered, or the synthesis of the miRNA may be promoted by the administration of the extracellular vesicles. In addition, the extracellular vesicles of the present disclosure may be incorporated and internalized into cells upon treatment with cells, and when the extracellular vesicles are incorporated and internalized into the cells, a clinically significant substance highly expressed in the extracellular vesicles may be effectively delivered to the cells to be highly expressed in the cells.

More specifically, the extracellular vesicles of the present disclosure may induce an increase in at least one miRNA selected from the group consisting of miR-19b-3p, miR-99a-5p, miR-100-5p, miR-101-3p, and miR-122-5p in patients with Moyamoya disease or Moyamoya syndrome.

The main genetic factor of Moyamoya disease or Moyamoya syndrome is a c.14429G>A (p.Arg4810Lys) variant of a Ring finger protein 213 (RNF213) gene. The variant is a mutation that is identified at a very low frequency in normal people, but is expressed at a particularly high rate in Moyamoya patients. The improvement of the decreased angiogenic ability and decreased cell proliferation ability induced by the genetic mutation and deletion of RNF213 may exhibit a therapeutic effect on Moyamoya disease or Moyamoya syndrome.

In one embodiment of the present disclosure, a Moyamoya disease model in which the RNF213 gene was knocked out was fabricated, and the therapeutic effect according to treatment of the extracellular vesicles of the present disclosure was confirmed. The extracellular vesicles of the present disclosure showed the effects of improving the contraction and reduction of blood vessels caused by the RNF213 mutation, increasing the reduced blood vessels, and increasing the number of vascular endothelial cells.

Therefore, the extracellular vesicles of the present disclosure may be used for improving the decreased angiogenic ability and cell proliferation ability of vascular endothelial cells caused by mutation or deletion of the RNF213 gene, and for promoting vascular anastomoses.

In particular, in the present disclosure, it was confirmed that these effects are associated with miRNAs that are abundantly included in the extracellular vesicles of the present disclosure and are down-expressed in Moyamoya patients, and through the administration of the extracellular vesicles of the present disclosure, an increase in at least one miRNA selected from the group consisting of miR-19b-3p, miR-99a-5p, miR-100-5p, miR-101-3p, and miR-122-5p may be induced in Moyamoya patients, and for example, an increase in miR-19b-3p or miR-101-3p may be induced.

Furthermore, in the present disclosure, it was confirmed that a Moyamoya-mimetic animal model was fabricated, a decrease in cerebral perfusion and morphological changes in blood vessels, which are representative symptoms of Moyamoya disease or syndrome, may be improved by the administration of the extracellular vesicles of the present disclosure. Specifically, the extracellular vesicles of the present disclosure effectively improved the stenosis occlusion of the internal carotid artery, the reduction in the diameter of the major cerebral blood vessels, the reduction in the collateral circulation blood vessels between the major cerebral blood vessels, and the resulting reduction in cerebral perfusion, which occurred in the Moyamoya-mimetic animal model.

More specifically, the extracellular vesicles of the present disclosure may increase the diameters of the internal carotid artery (ICA), the middle cerebral artery (MCA), the posterior communicating artery (Pcom), the anterior cerebral artery (ACA), and the basilar artery (BA) of the Circle of Willis (CoW), and the number of vascular anastomoses of the MCA and the ACA, and restore the diameter of the Circle of Willis to restore the vascular perfusion to a normal level. Therefore, the extracellular vesicles of the present disclosure may be used to induce vascular anastomosis, an increase in cerebral blood vessel diameter, or an increase in cerebral blood flow, and may be a specific therapeutic agent for Moyamoya disease or Moyamoya syndrome.

The pharmaceutical composition including the extracellular vesicles of the present disclosure may be administered to mammals such as mice, rats, livestock, and humans through various routes by all methods known in the art. All the methods of administration may be expected, and for example, the pharmaceutical composition may be administered by oral, rectal or intravenous, intramuscular, subcutaneous, intrauterine dural or intracerebroventricular injection.

In the present disclosure, the "Moyamoya disease" includes all Moyamoya diseases in which stenosis and occlusion of the cerebral artery progress bilaterally or unilaterally, and causes vascular occlusion, such as infection, vasculitis, autoimmune disease, Down syndrome, neurofibromatosis, or radiation therapy for brain tumors. Accordingly, Moyamoya disease may be separately distinguished from Moyamoya syndrome causing Moyamoya disease or also collectively referred to as Moyamoya disease. Therefore, the extracellular vesicles of the present disclosure may be used for preventing, treating, or alleviating Moyamoya disease or Moyamoya syndrome. At this time, the Moyamoya syndrome may refer to Moyamoya disease secondarily occurring by infection, vasculitis, autoimmune disease, Down syndrome, neurofibromatosis, or radiation therapy for brain tumors.

The composition including the extracellular vesicles of the present disclosure may be treated alone or simultaneously or sequentially with a conventional method used for treating or preventing Moyamoya disease or syndrome. For example, the composition may be administered before or after direct vascular anastomosis or indirect vascular anastomosis used for treating Moyamoya disease or syndrome, thereby further promoting angiogenesis and improvement of blood flow by the surgery.

The extracellular vesicles of the present disclosure are preferably extracellular vesicles derived from a three-dimensional spheroid-type cell aggregate prepared by (a) preparing a three-dimensional spheroid-type cell aggregate by 3-dimensionally (3D) static culturing stem cells in a microwell with a diameter of 200 to 800 µm and a depth of 100 to 1,000 µm; and (b) isolating extracellular vesicles from the three-dimensional spheroid-type cell aggregate.

**In** the present disclosure, the 3D culture means culturing in a three-dimensional arrangement in vitro, and unlike 2D culture, the cell growth in the 3D culture may allow cells to grow in all directions *in vitro* and may be more similar to an *in vivo* cell environment.

**In** the present disclosure, the 3D static-culture in step (a) may be performed by any 3D cell culture technique known in the art to which the present disclosure pertains, and may be a cell culture using, for example, microwell array culture, porous microsphere culture, hanging drop culture, low attachment plate culture, membrane-based cell-detachment culture, thermal lifting culture, centrifugation culture, semisolid medium culture, and the like. In the present disclosure, when the 3D culture of step (a) is static culture, there are advantages that it makes it easier to perform the culture without requiring devices necessary for shaking culture, and mass-culture is enabled in the Good manufacturing practices (GMP) manufacturing place.

In the present disclosure, the 3D static-culture in step (a) may be performed for 1 to 10 days, preferably 2 to 4 days. In the present disclosure, when the culture in step (a) is performed for 2 to 4 days, the viability of cells present in the three-dimensional spheroid-type cell aggregate is maintained at a high level, and the culture time is relatively short compared to a conventional process of preparing a three-dimensional spheroid-type cell aggregate, so that it is possible to rapidly prepare a three-dimensional spheroid-type cell aggregate and extracellular vesicles derived therefrom.

In the present disclosure, the 3D culture in step (a) may be performed by dispensing mesenchymal stem cells in a microwell at a density of 100 to 1,000 cells/well, preferably a density of 200 to 600 cells/well, and more preferably a density of 200 to 500 cells/well.

In the present disclosure, in the isolating of the extracellular vesicles in step (b), a sample including cells or a cell aggregate may be prepared by using a method selected from the group consisting of extrusion, sonication, cell lysis, homogenization, freeze-thawing, electroporation, chemical treatment, mechanical degradation, and treatment of physical stimuli externally applied to cells, and preferably isolated by a tangential flow filtration (TFF) method, but is not limited thereto.

In the present disclosure, the microwell may be coated with at least one selected from the group consisting of TMSPMA (3-(Trimetoxysily) propylmethacrylate), HEA (Hydroxyethyl acrylate), GMA (Glycidyl methacrylate), EGDMA (diethyleneglycol dimethacrylate), THFA (Tetrahydrofurfuryl acrylate), HMAA (Hydroxymethul acrylamide), PEA (Phenyl epoxyacrylate), HOFHA (6-Hydroxy-2,2,3,3,4,4,5,5-octafluoro), EOPT (Polyethoxylated(4)pentaerythritoltetraacrylate), HPA (Hydroxypropyl acrylate), BMA (Buthylmethacrlate), PETIA (Pentaerythritol triacrylate), HDDA (Hexan diol diacrylate), EGPEA (Ethyleneglycol phenyletheracrylate), BM (Benzylmethacrylate), HPPA (Hydroxyphenoxypropyl acrylate), BHPEA (2-(4-Benzoyl-3-hydroxyphenoxy)ethylacrylate), HEMA (Hydroxyethyl methacrylate), HPMA (N-(2-Hydroxypropyl) methacrylamide) and MPC (2-Methacryloyloxyethyl Phosphorylcholine Polymer), preferably MPC (2-Methacryloyloxyethyl Phosphorylcholine Polymer), but is not limited thereto.

The microwell of the present disclosure may have a diameter of 200 to 800 µm, preferably 300 to 800 µm, and more preferably 400 to 800 µm.

In addition, when the microwell may be a microwell having a flat structure without a depth, or a microwell with a depth, the microwell may have a structure of 100 to 1,000 µm, preferably 100 to 900 µm, and more preferably 200 to 900 µm.

The mesenchymal stem cells cultured by the structure may be maintained at a high survival rate even after the culture time has elapsed. Preferably, the production yield of cell aggregates may be increased by manufacturing a microarray including 1,000 to 100,000 microwells.

When the extracellular vesicles are prepared by the "method for preparing the extracellular vesicles derived from the three-dimensional spheroid-type cell aggregate" of the present disclosure, in addition to the preparation advantages of static culture, extracellular vesicles with improved clinical applicability, particularly therapeutic potential for Moyamoya disease or syndrome, may be mass-produced quickly and efficiently.

In the present disclosure, the cells may be used without limitation as long as the cells are cells capable of isolating extracellular vesicles, and may be cells isolated from natural organisms. In addition, the cells may be derived from any type of animal, including humans and non-human mammals, or plants and may be various types of immune cells, tumor cells, and stem cells, and preferably, the stem cells may be mesenchymal stem cells, pluripotent stem cells, induced pluripotent stem cells, or embryonic stem cells.

The extracellular vesicles prepared by the preparation method may be characterized by highly expressing at least one miRNA selected from the group consisting of miR-19b-3p, miR-99a-5p, miR-100-5p, miR-101-3p, and miR-122-5p, compared to naturally secreted extracellular vesicles or 2D cultured mesenchymal stem cell-derived extracellular vesicles.

Further, the present disclosure provides a pharmaceutical composition for preventing or treating Moyamoya disease or Moyamoya syndrome, including at least one miRNA selected from the group consisting of miR-19b-3p, miR-99a-5p, miR-100-5p, miR-101-3p and miR-122-5p.

The miR-19b-3p, miR-99a-5p, miR-100-5p, miR-101-3p and miR-122-5p may be included in various nucleic acid carriers known in the art or delivered in the form of a complex to increase delivery efficiency in vivo. For example, the miRNA may be included in a form included in a plasmid, a viral vector, or a non-viral carrier, and to this end, usable plasmids include, for example, pSilencer (Ambion), pSiEx (Novagen), siXpress (Takara Bio), pBLOCK-iTTM (Invitrogen), pcDNA3.1 (Invitrogen), pCEP4 (Invitrogen), SilenCircleTM (Allele), etc., but are not limited thereto. The viral carrier may be used with retrovirus vector, adenovirus vector, adeno-associated virus vector, vaccinia virus vector, lentivirus vector, herpes virus vector, alphavirus vector, EB virus vector, papillomavirus vector, and foamyvirus vectors, but is not limited thereto. In addition, as the non-viral carrier, a Mirus TrasIT-TKO lipophilic reagent as a delivery reagent, lipofectin, lipofectamine, cellfectin, G-fectin, cationic phospholipid nanoparticles, cationic polymers, cationic micelles, cationic emulsions or liposomes, ligand-DNA complexes, and gene guns may be used, but is not limited thereto. In the form of liposomes, the non-viral carrier is combined with lipids that exist as lamellar layers existing in an amphipathic agent, such as micelle, insoluble monolayer, liquid crystal, or aqueous solution. Lipids for the liposome formulation include monoglycerides, diglycerides, sulfatides, lysolecithin, lecithin phospholipids, saponins, bile acids, lipofectin, and the like, but are not limited thereto.

In addition, in the present disclosure, the miR-19b-3p, miR-99a-5p, miR-100-5p, miR-101-3p and miR-122-5p may be miRNAs in extracellular vesicles, and the extracellular vesicles may be extracellular vesicles derived from patient blood, preferably plasma. Accordingly, the miR-19b-3p, miR-99a-5p, miR-100-5p, miR-101-3p or miR-122-5p may be delivered in the pharmaceutical composition in the form of exosomes, microvesicles or extracellular vesicles. As used in the present disclosure, the "extracellular vesicles (EVs)" are classified into exosomes and microvesicles depending on a size, and the exosomes have the diameter of 30 to 150 nm, and the microvesicles have the size of 100 to 1,000 nm. The miRNAs included in the extracellular vesicles are protected from plasma RNase by a lipid bilayer, and thus show higher and more consistent miRNAs levels than cell-free miRNAs.

As a preferred example, the miR-19b-3p, miR-99a-5p, miR-100-5p, miR-101-3p and miR-122-5p of the present disclosure may be delivered to a subject in need of treatment in a form included in extracellular vesicles, but are not limited thereto, and for example, may be delivered in a form included in extracellular vesicles derived from the three-dimensional spheroid-type cell aggregate prepared by the preparation method of the present disclosure. The extracellular vesicles derived from the three-dimensional spheroid-type cell aggregate may be prepared by the preparation method including (a) preparing a three-dimensional spheroid-type cell aggregate by 3-dimensionally (3D) static culturing stem cells in a microwell with a diameter of 200 to 800 µm and a depth of 100 to 1,000 µm; and (b) isolating extracellular vesicles from the three-dimensional spheroid-type cell aggregate.

In addition, the miR-19b-3p, miR-99a-5p, miR-100-5p, miR-101-3p or miR-122-5p of the present disclosure may be formulated to use general ribonucleic acid intracellular delivery techniques known in the art, such as conjugating a biocompatible polymer such as polyethylene glycol to increase intracellular uptake, and the like, to enhance stability in the body.

The composition of the present disclosure may be used for the prevention or treatment of Moyamoya disease and Moyamoya syndrome, and may be used without limitation for Moyamoya disease that has progressed bilaterally or unilaterally, and Moyamoya syndrome which causes vascular occlusion and thus leads to Moyamoya disease. Preferably, the Moyamoya syndrome may be Moyamoya disease secondarily occurring by infection, vasculitis, autoimmune diseases, Down syndrome, neurofibromatosis or radiation therapy for a brain tumor.

The pharmaceutical composition of the present disclosure may further include suitable carriers, excipients, and diluents which are commonly used in the preparation of the pharmaceutical composition in addition to the active ingredient. The pharmaceutical composition of the present disclosure may further include other pharmaceutically active ingredients or active mixtures.

The pharmaceutical composition of the present disclosure may be formulated and used in the form of oral formulations such as powders, granules, tablets, capsules, suspensions, emulsions, syrups, aerosols, etc., external preparations, suppositories, and sterile injectable solutions according to conventional methods, respectively. The carrier, the excipient, and the diluent that may be included in the composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. When the composition is formulated, the formulations may be prepared by using diluents or excipients, such as a filler, an extender, a binder, a wetting agent, a disintegrating agent, and a surfactant, which are generally used. Solid formulations for oral administration include tablets, pills, powders, granules, capsules, and the like, and these solid formulations may be prepared by mixing at least one or more excipients, for example, starch, calcium carbonate, sucrose or lactose, gelatin, and the like. Further, lubricants such as magnesium stearate and talc are used in addition to simple excipients.

Liquid formulations for oral administration may correspond to suspensions, oral liquids, emulsions, syrups, and the like, and may include various excipients, for example, a wetting agent, a sweetener, an aromatic agent, a preservative, and the like, in addition to water and liquid paraffin which are commonly used as simple diluents. Formulations for parenteral administration include sterile aqueous solutions, non-aqueous solutions, suspensions, emulsions, lyophilized preparations, and suppositories. As the non-aqueous solution and the suspension, propylene glycol, polyethylene glycol, vegetable oil such as olive oil, injectable ester such as ethyl oleate, and the like may be used. As a base compound of the suppository, Witepsol, macrogol, Tween 61, cacao butter, laurinum, glycerogelatin, and the like may be used.

A preferable dose of the pharmaceutical composition of the present disclosure varies according to the condition and body weight of a patient, the degree of a disease, a drug form, and the route and period of administration, but may be properly selected by those skilled in the art. The administration may be performed once a day or several times a day. The dose does not limit the scope of the present disclosure in any aspect.

The pharmaceutical composition of the present disclosure may be administered to mammals such as mice, rats, livestock, and humans through various routes. All methods of administration may be expected, and for example, the pharmaceutical composition may be administered by oral, rectal or intravenous, intramuscular, subcutaneous, intrauterine dural or intracerebroventricular injection.

The definitions of terms for the excipients, binders, disintegrants, lubricants, flavors enhancers, flavoring agents, and the like of the present disclosure are described in literatures known in the art and include those with the same or similar functions.

Further, the present disclosure provides an *in vitro* composition for promoting vascular anastomosis of vascular endothelial cells induced by mutation or deletion of a Ring finger protein 213 (RNF213) gene, including extracellular vesicles derived from a three-dimensional spheroid-type cell aggregate prepared by (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

The *in vitro* composition of the present disclosure may be used for experimental purposes and may be a composition for treating isolated cells or tissues that have been identified to exhibit the characteristics of Moyamoya disease. The *in vitro* composition of the present disclosure may be a medium composition including the extracellular vesicles of the present disclosure, and the medium may include any medium known to those skilled in the art without limitation, for example, a medium including serum (e.g., fetal bovine serum, horse serum, and human serum). Media that may be used in the present disclosure may include, for example, RPMI series, EMEM, MEM, Iscove's MEM, 199 medium, CMRL 1066, RPMI 1640, F12, F10, DMEM, a mixture of DMEM and F12, Way-mo, McCoy's 5A, or media known in the art suitable for culturing cells exhibiting the characteristics of Moyamoya disease.

Further, the present disclosure provides a method for preparing a composition for preventing or treating Moyamoya disease or Moyamoya syndrome including extracellular vesicles derived from a three-dimensional spheroid-type cell aggregate, including (a) preparing a three-dimensional spheroid-type cell aggregate by 3 dimensionally (3D) static-culturing stem cells in microwells having a diameter of 200 to 800 µm and a depth of 100 to 1,000 µm; and (b) isolating extracellular vesicles from the three-dimensional spheroid-type cell aggregate.

According to the preparation method, the extracellular vesicles derived from the three-dimensional spheroid-type cell aggregate for preventing, alleviating, or treating Moyamoya disease or syndrome may be rapidly mass-produced in accordance with GMP standards.

Duplicated contents are omitted in consideration of the complexity of the present specification, and terms not defined otherwise in the present specification have the meanings commonly used in the art to which the present disclosure pertains.

Hereinafter, the present disclosure will be described in detail by Examples. However, the following Examples are just illustrative of the present disclosure, and the contents of the present disclosure are not limited to the following Examples.

### [Modes]

### Western blot

Cells and extracellular vesicles were dissolved in a radioimmunoprecipitation assay (RIPA) buffer (25 mM Tris-HCl, pH 7.6, 150 mM NaCl, 0.5% triton X-100, 1% Na-deoxycholate, 0.1% sodium dodecyl sulfate (SDS) and protease inhibitor). A total of 20 µg of proteins was separated by SDS-polyacrylamide gel electrophoresis and transferred to a nitrocellulose membrane (Bio-Rad, Hercules, CA, USA). Thereafter, the nitrocellulose membrane was cultured overnight at 4°C together with primary antibodies against histone H2A.Z, histone H3, lamin A/C, flotillin-1 (1 : 1,000, Cell signaling technology, Beverly, MA, USA) or calreticulin (1 : 1,000, ThermoFisher Scientific, Inc., Rockford, IL, USA). After washing with Tris-buffered saline-Tween 20, the nitrocellulose membrane was cultured for 2 hours together with a horseradish peroxidase (HRP)-conjugated secondary antibody (1 : 1,000, anti-rabbit, Cell Signaling Technology, Beverly, MA, USA). The proteins were detected using a chemiluminescent substrate from ThermoFisher Scientific, Inc. (Waltham, MA, USA). Labeled proteins were visualized via an X-ray film (Agfa, Mortsel, Belgium).

### ELISA

ELISA was performed with a commercial kit according to the manual of an individual manufacturer. The following ELISA kits were used: gentamicin (5111 GEN, EuroProxima, Arnhem, Nederland), bovine albumin (8100, Alpha Diagnostic, San Antonio, TX, USA), Hsp70 (Abcam, Cambridge, UK), CD63, CD9 and CD81 (System Biosciences, Palo Alto, CA), USA), histone H2A.Z. (Mybiosource, San Diego, CA, USA), calreticulin (Mybiosource) and cytochrome C (ThermoFisher Scientific, Inc.). All the kits included standard proteins; therefore, the amounts of proteins and extracellular vesicles were determined based on a standard curve of each kit.

### qPCR

RNA was extracted from EVs using Trizol^{™} according to the instructions of the manufacturer and RNA was quantified by a nanodrop. The RNA was made into cDNA through a reverse transcription (RT) process, and real-time PCR was performed according to the manual of the manufacturer using Taqman probes suitable for each miRNA and mRNA.

### Example 1. Isolation of extracellular vesicles through 3D culture of mesenchymal stem cells

### 1.1 Preparation of mesenchymal stem cells

Wharton's Jelly-derived mesenchymal stem cells (hereinafter, WJ-MSC, Samsung medical center, Seoul, Korea) at passage 5 stage were received and cultured in a 37°C, 5% CO₂ incubator. A growth medium was used with an α-modified eagle's medium (α-MEM, GIBCO, NY, USA) including 10% fetal bovine serum (FBS) (GIBCO, NY, USA) and 50 µg/mL gentamicin (GIBCO, NY, USA). WJ-MSCs of passage 6 stage were used to prepare a 3D spheroid-type cell aggregate.

### 1.2 Production of 3D spheroid-type cell aggregate culture medium

The WJ-MSCs of passage 6 stage prepared in Example 1.1 were washed with PBS, treated with trypsin (TrypLETM Express, GIBCO, NY, USA) and reacted in a CO₂ incubator for 5 minutes. Thereafter, a new serum-free medium was added to neutralize trypsin, and the cells were recovered to obtain a cell pellet using a centrifuge. Then, a new serum-free medium was added to prepare a cell suspension, and the cells were counted. After cell counting, 60 ml of the cell suspension was uniformly dispensed in a microarray including 69,000 microwells coated with 2-methacryloyloxyethyl phosphorylcholine polymer (MPC) with the diameter and depth of 500 µm × 200 µm, respectively, to be a density of 400 cells/well, and maintained in a static state to induce the spontaneous formation of spheroid-type cell aggregates, and cultured in a CO₂ incubator at 37°C for a total of 4 days to prepare a 3D spheroid-type cell aggregate culture medium (hereinafter, referred to as a 3D-static-spheroid culture medium).

### 1.3 Isolation of extracellular vesicles derived from 3D spheroid-type cell aggregate

The 3D-static-spheroid culture medium prepared in Example 1.2 was collected, centrifuged at 2,500 g for 10 minutes, and filtered with a 0.22 µm syringe filter to remove cell debris. Thereafter, the 3D-static-spheroid culture medium passed through 300 kDa of a hollow fiber membrane (Pall, NY, USA) using a tangential flow filtration (TFF) system to remove proteins, and extracellular vesicles were first isolated and purified once more with physiological saline to obtain high-purity 3D-static-spheroid-derived extracellular vesicles (hereinafter, 3D-static-spheroid EVs) of the present disclosure.

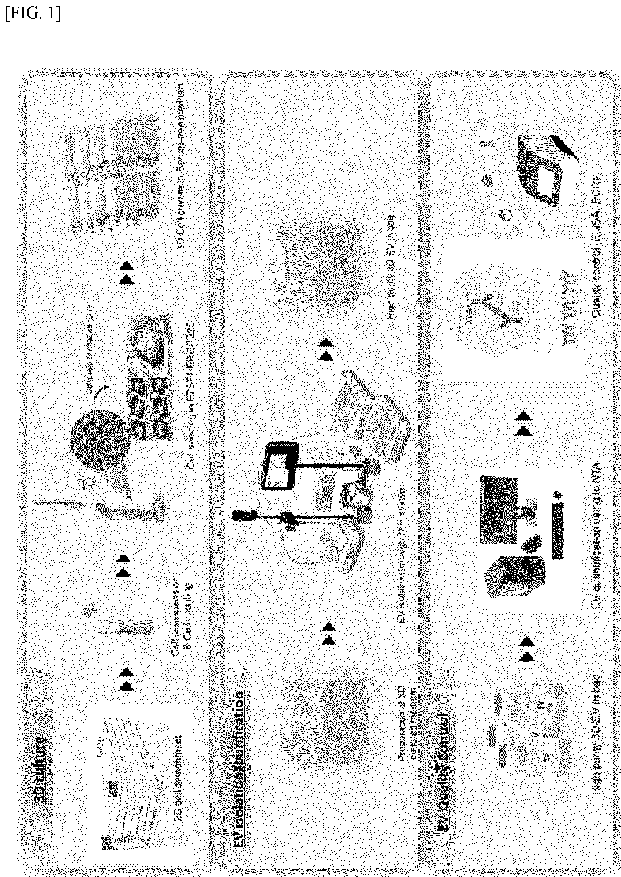

The processes of Examples 1.1 to 1.3 were schematically illustrated in FIG. 1.

### Example 2. Analysis of characteristics of 3D spheroid-type cell aggregate

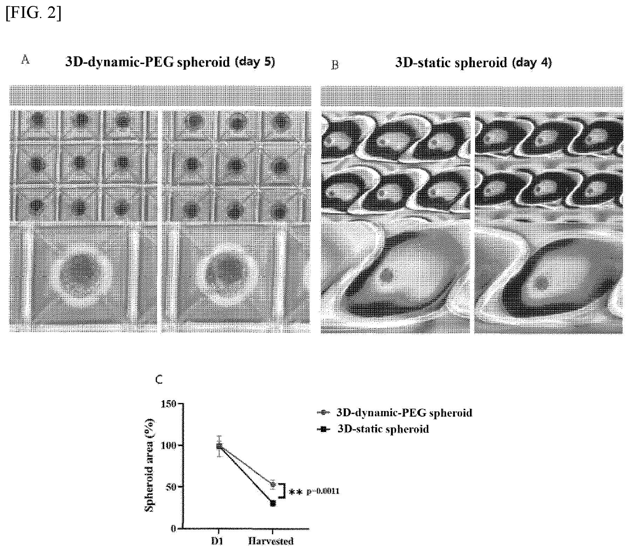

The characteristics of the 3D-spheroid-type cell aggregate (hereinafter, referred to as 3D-static-spheroid) present in the 3D-static-spheroid culture medium prepared in Example 1.2 were analyzed. As an experimental group, the 3D-static-spheroid culture medium prepared in Example 1.2 was used, and as a comparative group, a 3D mesenchymal stem cell spheroid-type cell aggregate (hereinafter referred to as 3D-dynamic-PEG spheroid) culture medium prepared by culturing mesenchymal stem cells for 5 days was used according to a dynamic 3D cell culture method disclosed in Korean Registered Patent (Application No. 10-2016-0053026, method for producing stem cell-derived extracellular vesicles). With an optical microscope, microwells containing each culture medium were observed, which were illustrated in FIGS. 2A and 2B, and changes in spheroid-type cell aggregate area after culture compared to the initial culture was illustrated in FIG. 2C.

As illustrated in FIG. 2, as a result of comparing the areas of the 3D-static-spheroid of the experimental group and the 3D-dynamic-PEG spheroid of the comparative group, depending on a characteristic in which cells were densely condensed to form spheroids, compared to 3D-dynamic-PEG spheroids, 3D-static-spheroids showed a statistically significant decrease in area compared to spheroids at the initial culture (p = 0.0011).

### Example 3. Size analysis of 3D spheroid-type cell aggregate

The size distributions of the 3D-static-spheroid prepared in Example 1.2 and the 3D-dynamic-PEG spheroid prepared in Example 3 were measured, and based on the measured size distribution data, dispersion coefficient, skewness and kurtosis were analyzed.

The skewness is a parameter capable of determining the inclined direction and degree of the distribution from the trend of a median value, and indicates a distribution with a longer tail to the right as closer to 1, and indicates a distribution with a longer tail to the left as closer to - 1. In the case of a normal distribution, the skewness is 0.

The kurtosis is a parameter that indicates the degree of sharpness of the data distribution, and if the value is positive, the kurtosis means that a relatively large number of data points are accumulated in the central part, and if the value is negative, the kurtosis means that relatively few data are accumulated in the central part. In the case of a normal distribution, the kurtosis is 0.

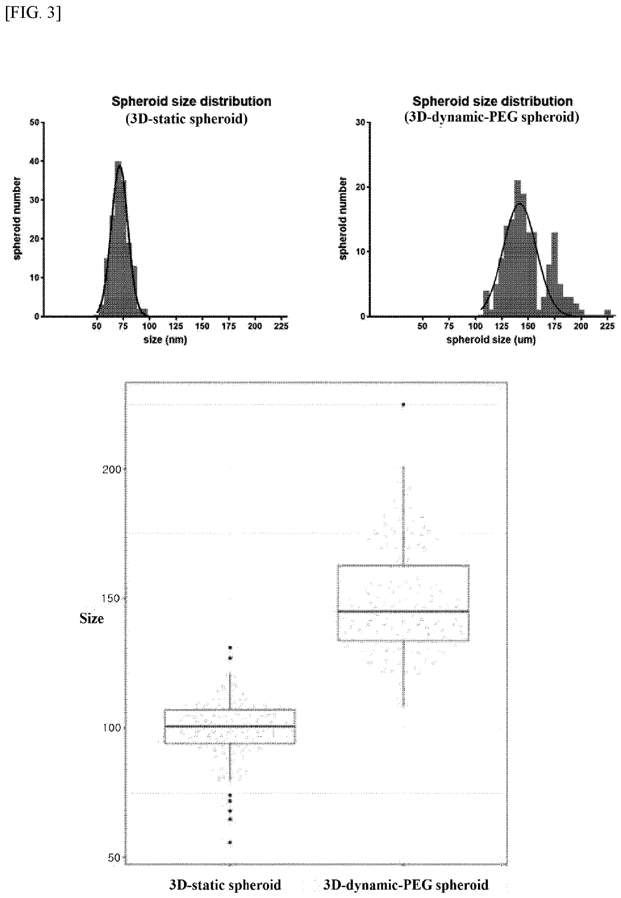

The measured size distribution data was illustrated in FIG. 3, and the results of analyzing the data were illustrated in Table 1.

**[Table 1]**

| | 3D-dynamic-PEG spheroid | 3D-static-spheroid |
|---|---|---|
| The number of spheroids | 154 | 155 |
| Size Range | 108.4 - 225.0 µm | 55.0 - 95.0 µm |
| Size (mean ± SD) | 148.66 ± 20.89 µm | 74.43 ± 7.756 µm |
| Coefficient of variance | 14.1% | 9.59% |
| Skewness | 0.694 ± 0.195 | -0.745 ± 0.195 |
| Kurtosis | 0.350 ± 0.389 | 1.799 ± 0.389 |

As illustrated in FIG. 3 and Table 1, it was confirmed that the average size of the 3D-static-spheroids was 74.43 µm, and the coefficient of variance (CV) was 9.59%. In contrast, it was confirmed that the 3D-dynamic-PEG spheroids had an average size of 148.66 µm and a coefficient of variance (CV) of 14.1%.

To compare and test the measured coefficients of variance, as a result of performing a Feltz and Miller's (1996) asymptotic test, a p value was calculated as 0.01765604, and as a result of performing a Krishnamoorthy and Lee's (2014) modified signed-likelihood ratio test, the p value was calculated as 0.01853969. Accordingly, in both the tests, it was confirmed that the size distributions of 3D-static-spheroids and 3D-dynamic-PEG spheroids showed statistically significant different patterns.

As a result of comparing the size distributions of the 3D-static-spheroids and the 3D-dynamic-PEG spheroids, it was confirmed that the kurtosis value of the size distribution of the 3D-static-spheroids was relatively large, and the size distribution of the 3D-static-spheroids of the present disclosure showed a tendency to be concentrated in the median values. From these results, it was confirmed that when the 3D spheroids were prepared by the method for preparing the 3D-static-spheroids of the present disclosure, it was possible to prepare 3D spheroids having a relatively constant size.

### Example 4. Morphological analysis of extracellular vesicles derived from 3D spheroid-type cell aggregate

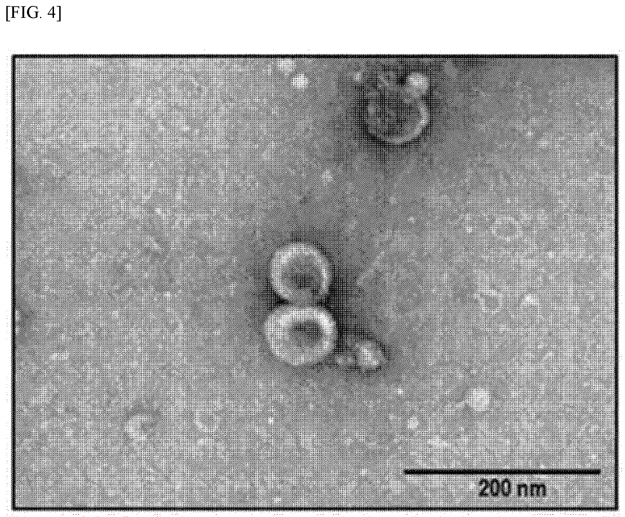

In order to observe the morphology of the 3D-static-spheroid EVs isolated in Example 1.3, the 3D-static-spheroid EVs were photographed using a transmission electron microscopy (TEM). Specifically, the 3D-static-spheroid EVs were fixed with 1% OsO₄ dissolved in a 0.1 M phosphate buffer (PB) for 2 hours. The EM grids were adsorbed onto the droplets of extracellular vesicles with a formvar side facing downward for 1 minute. Thereafter, the EM grids were blotted with filter paper and reacted with 2% uranyl acetate for 15 seconds. Excessive uranyl acetate was removed, and the EM grids were observed using a TEM (JEM-1011, JEOL, Japan), and the observed image was illustrated in FIG. 4.

As illustrated in FIG. 4, it was confirmed that the 3D-static-spheroid EV had a round shape, which was a typical shape of extracellular vesicles.

### Example 5. Concentration and size analysis of extracellular vesicles derived from 3D spheroid-type cell aggregate

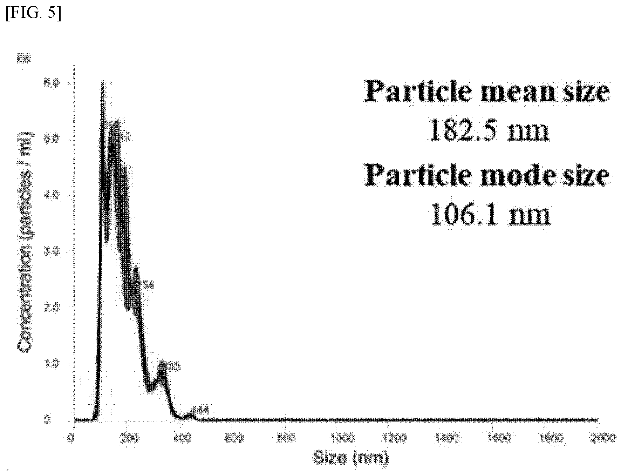

In order to confirm the concentration and size distribution of 3D-static-spheroid EVs isolated in Example 1.3, nanoparticle tracking analysis (NTA) was performed using NanoSight NS300 (Malvern, Worcestershire, UK). For optimal analysis, the 3D-static-spheroid EVs were pre-diluted in a vesicle-free phosphate buffer (PBS). The average size and concentration (particle/mL) were calculated by integrating three records, and the results were illustrated in FIG. 5.

As illustrated in FIG. 5, it was confirmed that the average particle diameter of the 3D-static-spheroid EV was 182.5 nm and the mode diameter was 106.1 nm.

### Example 6. Analysis of expression markers of extracellular vesicles derived from 3D spheroid-type cell aggregate

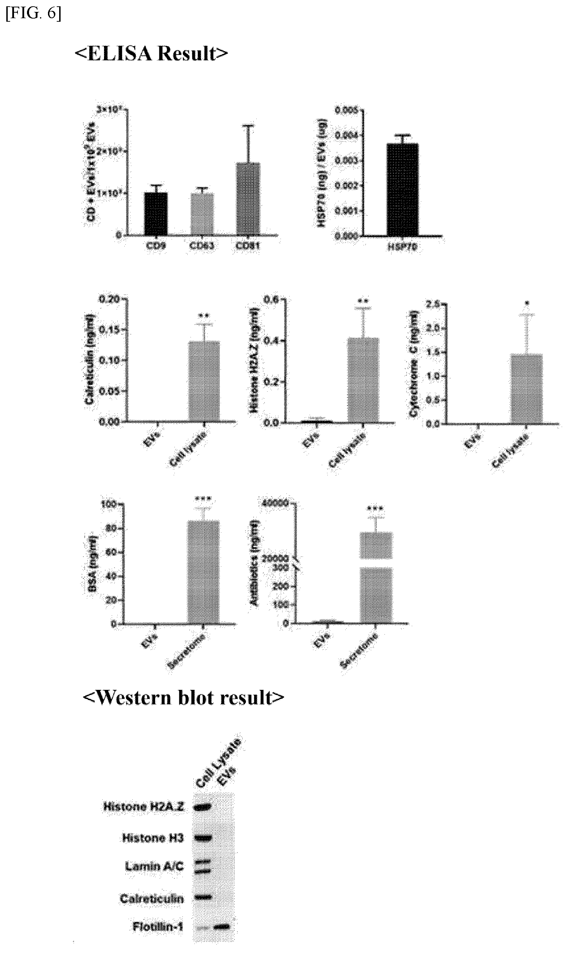

Experiments were performed to confirm expression markers of the 3D-static-spheroid EVs isolated in Example 1.3. A cell lysate and a secretome were used as a control. The cell lysate was prepared by a method of washing the 3D-static-spheroids with PBS, treating the 3D-static-spheroids with trypsin to collect the cells, and obtaining a cell pellet using a centrifuge of the collected cells. The secretomes were prepared by a method of obtaining the cell pellet, isolating EVs from a supernatant of the culture medium through the process of Example 1.3, and obtaining the remaining culture secretomes. For marker analysis, extracellular vesicle-specific positive markers CD9, CD63, CD81 and HSP70 were quantified using ELISA, and specific contaminant protein markers such as calreticulin, histone H2A.Z, cytochrome C, albumin, and antibiotics were quantified. In addition, histone H2A.Z, histone H3, lamin A/C, and calreticulin, which were specific contaminant protein markers of extracellular vesicles, were quantified using Western blot, and a positive marker of extracellular vesicles, flotillin-1 was quantified. The ELISA analysis result and the Western blot result were illustrated in FIG. 6.

As illustrated in FIG. 6, it was confirmed that 3D-static-spheroid EVs expressed all of the extracellular vesicle-specific positive markers CD9, CD63, CD81 and HSP70, and calreticulin, histone H2A.Z, histone H3, cytochrome C, albumin (BSA, bovine serum albumin), lamin A/C, and antibiotics, which were contaminant protein markers highly expressed in the cell lysates and the secretomes, were not almost expressed. In particular, it was confirmed that Flotillin-1 as a positive marker for extracellular vesicles, which was expressed very low in the cell lysate, was relatively highly expressed in 3D-static-spheroid EVs.

### Example 7. Analysis of production of extracellular vesicles derived from 3D spheroid-type cell aggregate

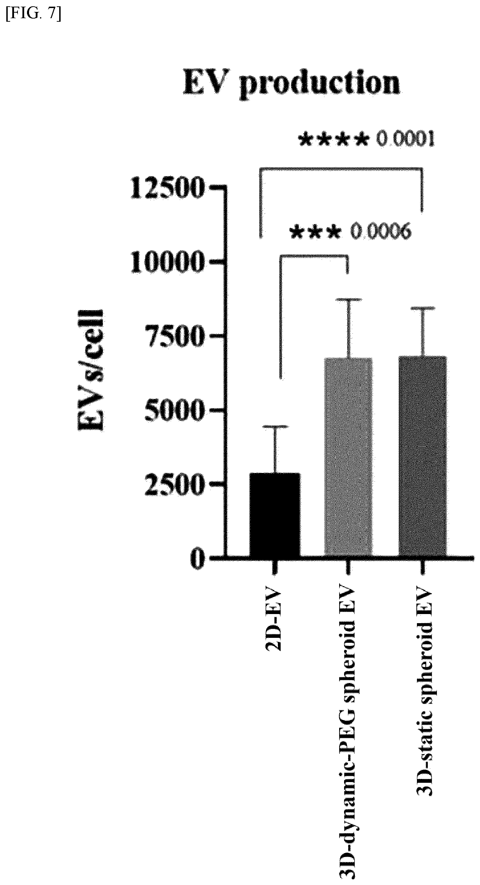

The experiment was performed to compare the productions of the 3D-static-spheroid EVs, which were the extracellular vesicles prepared by the preparation method of Example 1, the extracellular vesicles (3D-dynamic-PEG spheroid EVs) isolated from the 3D-dynamic-PEG spheroids of Example 2, and extracellular vesicles (2D-EVs) isolated from stem cells cultured by a conventional 2D culture method. The 2D-EVs were prepared by the following process. The stem cells cultured for 3 days in a cell stack were washed with PBS, replaced with a serum-free medium, and further cultured for 2 days. The culture medium was recovered, and the cell debris was continuously removed using centrifugation and a 0.2 µm filter. Thereafter, the culture medium passed through a hollow fiber membrane using a TFF system to remove proteins, and the EVs were first isolated, and purified once more with physiological saline to obtain high-purity 2D-EVs. The productions per cell derived from the prepared 3D-static-spheroid EVs, 3D-dynamic-PEG spheroid EVs, and 2D-EVs were compared and illustrated in Table 2 and FIG. 7.

**[Table 2]**

| | 2D-EV | 3D-dynamic-PEG spheroid EV | 3D-static-spheroid EV |
|---|---|---|---|
| Production (EVs/cell) | 2437 EVs/cell | 6791 EVs/cell | 6840 EVs/cell |

### Example 8. Verification of 3D-static-spheroid EV effect according to preparation conditions

### 8.1 Experimental methods and conditions

Through Examples above, the excellent effect of the 3D-static-spheroid EVs of the present disclosure was confirmed. The preparation method of Example 1 was performed in the same manner, but to confirm whether the same effect was observed even in EVs prepared under different microwell specifications and cell number conditions, the cells were incubated by changing the cell count standard per microwell from 400 cells/well to 200 cells/well, or changing the microwell specification from 500 µm × 200 µm in diameter and depth to 500 µm × 600 µm, or a flat well with a diameter of 800 µm and no depth.

The experimental conditions changed from the preparation method of Example 1 were shown in Table 3 below.

**[Table 3]**

| | Experimental Example 1 | Experimental Example 2 | Experimental Example 3 |
|---|---|---|---|
| Number of cells per microwell (cell/microwell) | 400 | 400 | 200 |
| Diameter of microwell (µm) | 500 | 800 | 4-500 |
| Depth of microwell (µm) | 600 | / | 200 |

The WJ-MSCs of passage 6 stage prepared in Example 1.1 were washed with PBS, treated with trypsin (TrypLETM Express, GIBCO, NY, USA) and reacted in a CO₂ incubator for 5 minutes. Thereafter, a new serum-free medium was added to neutralize trypsin, and the cells were recovered to obtain a cell pellet using a centrifuge. Then, a new serum-free medium was added to prepare a cell suspension, and the cells were counted. After counting the cells, the cells were uniformly dispensed into microwells under the conditions shown in Table 3 above, maintained in a static state to induce spontaneous formation of spheroid-type cell aggregates, and incubated in a CO₂ incubator at 37°C for a total of 4 days to prepare a 3D spheroid-type cell aggregate culture medium.

### 8.2 Confirmation of 3D spheroid-type cell aggregate morphology

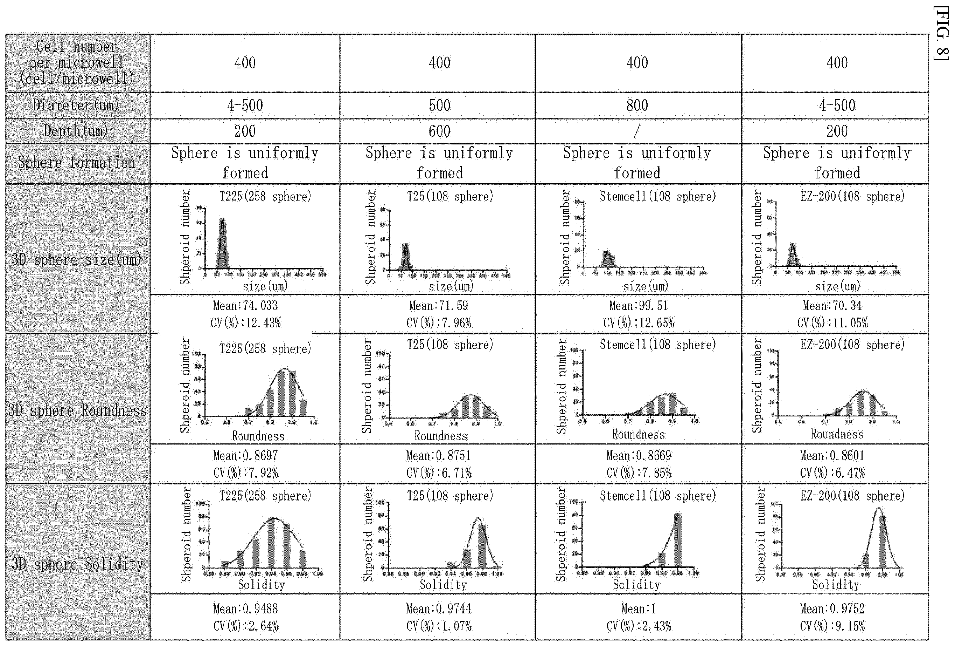

It was confirmed that spheroids were formed uniformly in the same manner as in Example 1 under the conditions of Experimental Examples 1 to 3, and the 3D spheroid-type cell aggregate culture medium was recovered to obtain spheroid-derived extracellular vesicles by the method of Example 1.3. The size distribution of the obtained extracellular vesicles was measured, and Roundness and Solidity of the spheroids were additionally confirmed, and the results were illustrated in FIG. 8.

As illustrated in FIG. 8, it was confirmed that the sizes of the 3D spheroid-type cell aggregates prepared in Experimental Examples 1 to 3 were within the range of 55 to 131 µm, which was the size range of the cell aggregate prepared in Example 1, and it was confirmed that the average values of the size distribution were 70.34 to 99.51 µm. In addition, as a result of confirming the Roundness and Solidity, it was confirmed that similarly to the average Roundness value of 0.8697 (CV 2.64%) of the 3D spheroid-type cell aggregate of Example 1, the spheroid-type cell aggregates of Experimental Examples 1 to 3 also had Roundness values of 0.8751, 0.8669, and 0.8601, respectively. In addition, it was confirmed that similarly to the average Solidity value of 0.9488 (CV 2.64%) of the 3D spheroid-type cell aggregate of Example 1, the spheroid-type cell aggregates of Experimental Examples 1 to 3 also had the average values of 0.9744, 1, and 0.9752, respectively.

These results showed that even if the number of cells per microwell was changed to 200 or 400 and the diameter of the microwell was changed to 400 to 800, the 3D spheroid-type cell aggregate with a similar shape may be effectively formed through the method of Example 1.

### 8.3 Comparison of miRNA expression patterns of 3D spheroid-type derived EVs

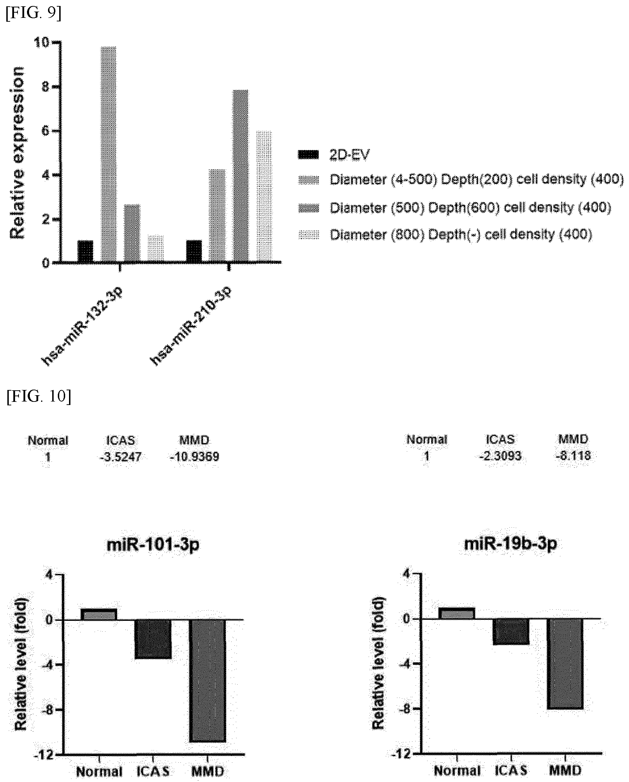

Since it was confirmed that spheroid-type cell aggregates having a similar shape to Example 1 may be prepared even under the conditions of Experimental Examples 1 to 3, additionally, extracellular vesicles were isolated and obtained from the spheroid-type cell aggregates by the method of Example 1.3, and it was confirmed whether the isolated and obtained EVs also showed the same miRNA expression patterns. Comparison of the miRNA expression patterns was confirmed using extracellular vesicles derived from the spheroid-type cell aggregates prepared by the methods of Experimental Examples 1 and 2 under different microwell conditions. The miRNA expression analysis was confirmed through a qPCR method. The results were shown in FIG. 9. The miRNA expression pattern was compared with that of the 2D-EV prepared in Example 7.

As illustrated in FIG. 9, it was confirmed that even if the microwell conditions were changed to 500 and 800 µm in diameter, in the same manner as the EVs prepared in Example 1, the expression of miR-132 and miR-210 was significantly increased compared to conventional 2D-EVs. These results showed that EVs derived from the three-dimensional spheroid-type cell aggregates obtained by the method of the present disclosure using microwells with a diameter of 200 to 800 µm may commonly exhibit miRNA marker expression characteristics. Therefore, these EVs may all be referred to as 3D-static-spheroid EVs.

### Example 9. Identification of candidate miRNAs for Moyamoya therapy

After receiving the consent from clinical trial participants (Clinicaltrials.gov. Unique identifier: NCT02743052), plasma samples were obtained from 10 patients with Moyamoya (MMD), 10 normal controls, and 10 patients with intracranial arteriosclerosis (ICAS).

The levels of miRNAs in the pooled samples were identified in microvesicles isolated from the samples using a microarray mRNA experiment. Specifically, miRNA profiles in sample microvesicles were measured using the Human whole-miRNome Array and a miScript SYBR green PCR kit. 4 µl RNA was reverse-transcribed into cDNA using a miScript II RT kit. A PCR template was used at a 1 : 20 dilution of a RT product, and qRT-PCR was performed in 384 wells including synthetic miRNAs miRTC, a positive PCR control group, and a housekeeping reference using the ABI 7900HT Real-Time PCR System. Raw data were normalized to the reference miRNA included in all plates and normalized to the Global CT mean to calculate Cq values. The miRNA patterns of plasma-derived microvesicles from the normal group, ICAS, and Moyamoya patients were compared, and among them, miRNAs that were specifically differentially up- or down-regulated in Moyamoya patients were identified compared to the normal group and ICAS. In addition, plasma was isolated from the blood of 4 patients with Moyamoya disease (MMD) and 4 normal controls, and extracellular vesicles were isolated by ultracentrifugation. RNA was isolated from the isolated extracellular vesicles and small RNA sequencing was performed.

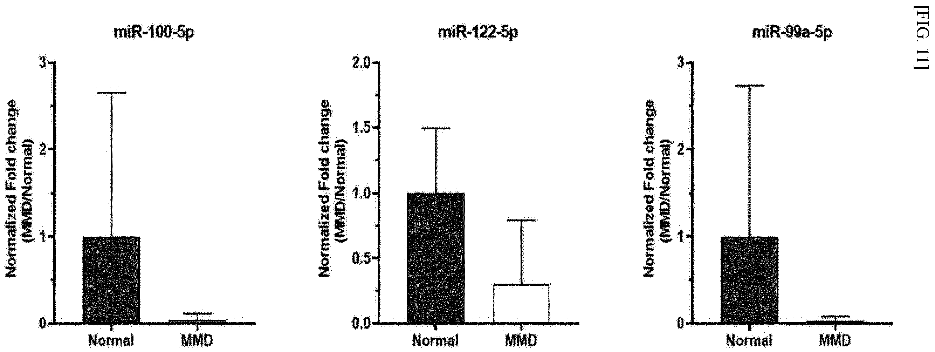

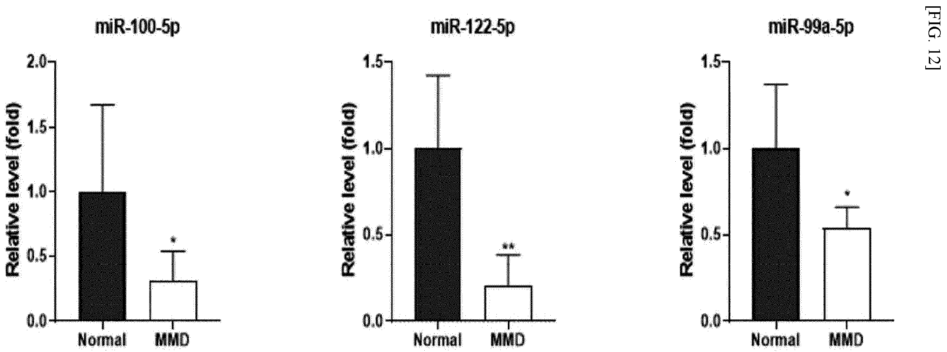

As a result, it was confirmed that miR-19b-3p (MIMAT0000074, UGUGCAAAUCCAUGCAAAACUGA (SEQ ID NO: 1)), miR-101-3p (MIMAT0000099, UACAGUACUGUGAUAACUGAA (SEQ ID NO: 2)), miR-100-5p (MIMAT0000098, AACCCGUAGAUCCGAACUUGUG (SEQ ID NO: 3)), miR-122-5p (MIMAT0000421, UGGAGUGUGACAAUGGUGUUUG (SEQ ID NO: 4)), and miR-99a-5p (MIMAT0000097, AACCCGUAGAUCCGAUCUUGUG (SEQ ID NO: 5)) were specifically down-regulated in Moyamoya patients compared to the normal group, and the results were illustrated in FIGS. 10 and 11. In addition, quantitative PCR was performed using a taqman probe for the miR-100-5p, miR-122-5p, and miR-99a-5p, which were confirmed to be decreased in Small RNA seq, and the results were illustrated in FIG. 12.

As illustrated in FIGS. 10, 11, and 12, it was confirmed that miR-101-3p, miR-19b-3p, miR-100-5p, miR-122-5p, and miR-99-5p were significantly down-expressed in microvesicles derived from plasma of Moyamoya patients, unlike in the normal group. Through these results, it was confirmed that plasma microvesicle-derived miR-101-3p, miR-19b-3p, miR-100-5p, miR-122-5p, and miR-99-5p may be therapeutic candidates for Moyamoya disease. The Accession numbers and sequence information of miR-19b-3p, miR-99a-5p, miR-100-5p, miR-101-3p and miR-122-5p of the present disclosure were shown in Table 4.

**[Table 4]**

| **Name** | **Accession number** | **Sequence** | **Sequence number** |
|---|---|---|---|
| hsa-miR-19b-3p | MIMAT0000074 | UGUGCAAAUCCAUGCAAAACUGA | 1 |
| hsa-miR-101-3p | MIMAT0000099 | UACAGUACUGUGAUAACUGAA | 2 |
| hsa-miR-100-5p | MIMAT0000098 | AACCCGUAGAUCCGAACUUGUG | 3 |
| hsa-miR-122-5p | MIMAT0000421 | UGGAGUGUGACAAUGGUGUUUG | 4 |
| hsa-miR-99a-5p | MIMAT0000097 | AACCCGUAGAUCCGAUCUUGUG | 5 |

### Example 10. Confirmation of Moyamoya-related miRNA expression in 3D-static-spheroid EVs

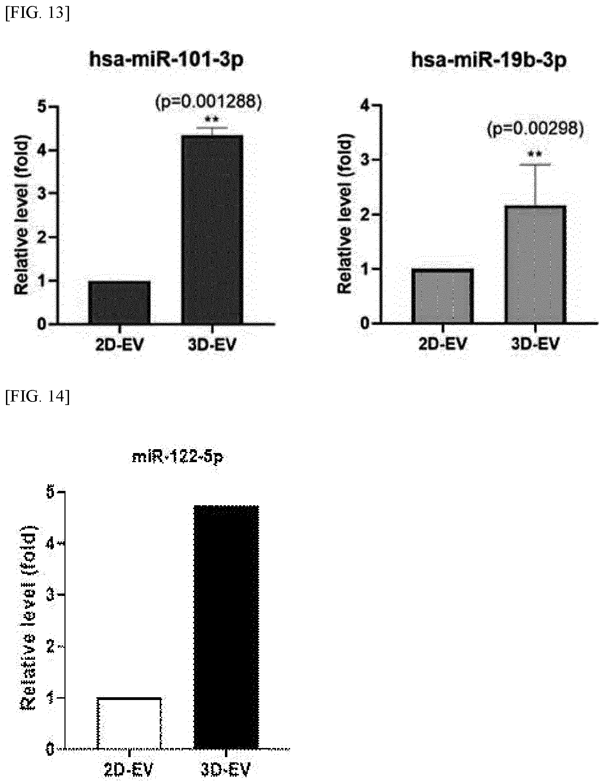

The expression of miR-101-3p, miR-19b-3p, miR-100-5p, miR-122-5p and miR-99-5p was confirmed in 3D-static-spheroid EVs, which were extracellular vesicles prepared by the preparation method of Example 1, and compared with the expression of conventional 2D-EVs obtained by the method of Example 7 or compared using U6 snRNA as an internal control group. The miRNA expression level measurement was confirmed through qPCR using a tagman probe, and the results were illustrated in FIGS. 13 and 14.

As a result, it was confirmed that the 3D-static-spheroid EV of the present disclosure showed a relatively low Ct value to have high miRNA expression, and as illustrated in FIGS. 13 and 14, as the qPCR results, it was confirmed that the expression of miR-101-3p, miR-19b-3p, and miR-122-5p was significantly high in the 3D-static-spheroid EVs of the present disclosure compared to conventional 2D-EVs.

That is, through the results, it was confirmed that the 3D-static-spheroid EVs were EVs with novel miRNA expression characteristics compared to conventional 2D-EVs, and in particular, since the 3D-static-spheroid EVs included a high ratio of all of miR-101-3p, miR-19b-3p, miR-100-5p, miR-122-5p, and miR-99-5p, which were down-expressed in microvesicles of Moyamoya patients, it was confirmed that the 3D-static-spheroid EVs have the possibility of becoming a therapeutic agent for Moyamoya disease.

### Example 11. Confirmation of Moyamoya therapeutic effect by 3D-static-spheroid EV treatment - in vitro

To confirm whether 3D-static-spheroid EVs including a high ratio of miRNAs down-regulated in the microvesicles of Moyamoya patients had a Moyamoya therapeutic effect, a Moyamoya disease model using vascular endothelial cells was prepared and its effect was confirmed.

The RNF213 gene, known as a major genetic factor of Moyamoya disease, was knocked out to produce RNF213 silenced cells, the corresponding model was treated with the 3D-static-spheroid EV, and then a Moyamoya disease therapeutic effect was confirmed.

To measure the cell phenotype and gene expression due to RNF213 siRNA, 1.5 × 10⁵ HUVECs were inoculated in a 6-well culture plate. The cells were transfected the next day using RNAimax from Invitrogen according to the manufacturer's protocol. The cells were transfected for 6 hours in a CO₂ humidified chamber, and the siRNA used for transfection was used with a siRNA mixture of two sequences targeting RNF213, and a negative control siRNA fabricated so as not to target any gene was used as a control. After transfection of each siRNA into the cells at 60 nM, the medium was replaced with an antibiotic-free medium. The transfected HUVECs were treated with 4.5 × 10⁸ EVs, and 16 hours after EV treatment, the HUVECs were used for a phenotypic experiment.

The siRNA and RNF213 siRNA sequences used for a control experiment were shown in Table 5 below.

**[Table 5]**

| No. | Genes | Sense | Antisense |
|---|---|---|---|
| 1 | Control siRNA | UUCUCCGAACGUGUCACGU (SEQ ID NO: 6) | ACGUGACACGUUCGGAGAA (SEQ ID NO: 7) |
| 2 | RNF213 siRNA #1 | CAAAAUGGGACAGCAAUAU (SEQ ID NO: 8) | AUAUUGCUGUCCCAUUUUG (SEQ ID NO: 9) |
| 3 | RNF213 siRNA #2 | CAGAUGUCCAGGAAGUGAA (SEQ ID NO: 10) | UUCACUUCCUGGACAUCUG (SEQ ID NO: 11) |

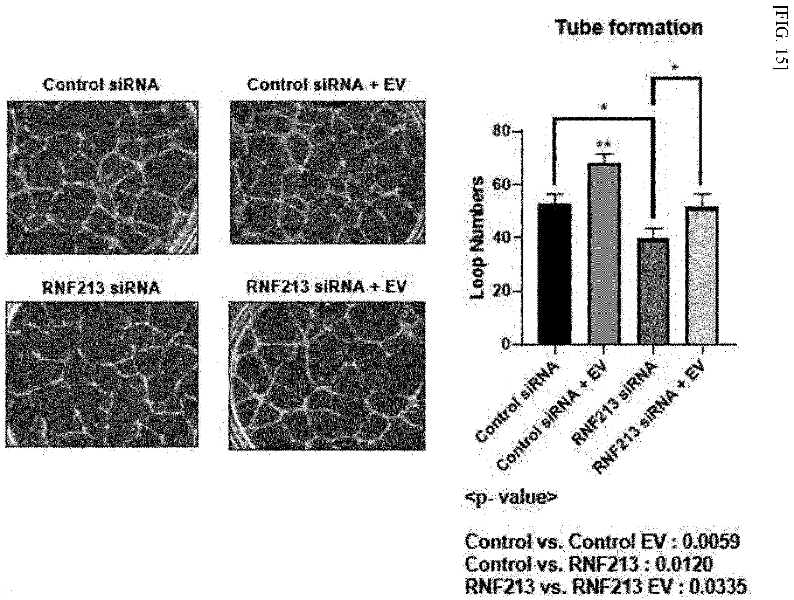

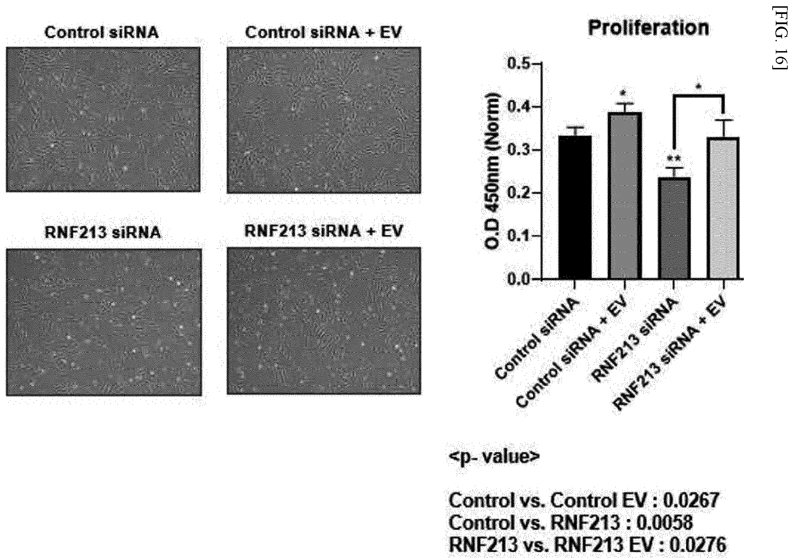

Specifically, in order to confirm a tube formation effect, HUVECs were cultured in an M199 medium (Gibco) supplemented with 20% FBS, 5 U/mL heparin, and 3 ng/mL bFGF. The cells were inoculated on a growth factor-reduced Matrigel Matrix (BD Bioscience, MA, USA) from µ-Slides Angiogenesis (ibidi, Graefelfing, Germany) at a density of 1.7 × 10⁴ cells, and the tube was formed for 4 hours in a humidifying chamber under a 37°C and 5% CO₂ environment. Images were taken on a phase-contrast microscope (Olympus), and the number of tubular structures was quantified in a microscopic field (4× magnification) using ImageJ software. The changes in angiogenic ability and cell proliferation ability of vascular endothelial cells were confirmed according to RNF213 siRNA treatment and 3D-static-spheroid EV treatment, and the results were illustrated in FIGS. 15 and 16. As illustrated in FIGS. 15 and 16, the RNF213 siRNA treatment group showed a decrease in blood vessels compared to the control group. Thereafter, it was confirmed that when the 3D-static-spheroid EV of the present disclosure was treated, the morphological changes of blood vessels due to RNF213 mutation were improved, the decreased blood vessels were increased again, and the number of vascular endothelial cells was increased.

These results show that the 3D-static-spheroid EV may be used as a therapeutic agent for Moyamoya disease.

### Example 12. Confirmation of changes in Moyamoya-related miRNA expression according to treatment with 3D-static-spheroid EV

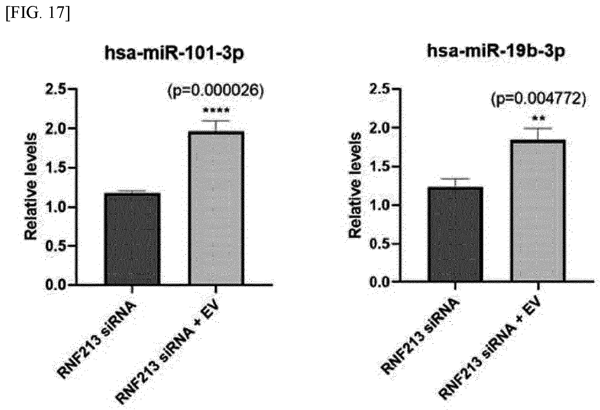

Through Example 11, it was confirmed that the 3D-static-spheroid EV improved morphological changes of blood vessels and symptoms of decreased blood vessels due to RNF213 mutation in a Moyamoya disease model and restored cell proliferation ability. Therefore, an experiment was performed to confirm whether the effect of the 3D-static-spheroid EV was achieved by up-regulating the expression of miRNA that had been decreased in Moyamoya patients. Specifically, after vascular endothelial cells were treated with RNF213 siRNA in Table 5, the expression changes of miR-101-3p and miR-19b-3p were confirmed. In addition, after treating siRNA, the 3D-static-spheroid EV was treated to confirm whether the expression pattern of the miRNA changed, and the results of measuring the relative expression levels were illustrated in FIG. 17.

As illustrated in FIG. 17, it was confirmed that the expression of miR-101-3p and miR-19b-3p in vascular endothelial cells was significantly increased as a result of treating the 3D-static-spheroid EV. This result shows that the therapeutic effect on Moyamoya disease by treatment of the 3D-static-spheroid EV is related to an increase in miRNAs due to miRNAs abundantly expressed in the 3D-static-spheroid EV. That is, it was confirmed that when the 3D-static-spheroid EV of the present disclosure was treated, the down-expressed miRNA was increased in Moyamoya patients compared to the normal group to exhibit a therapeutic effect on Moyamoya disease.

### Example 13. Confirmation of Moyamoya therapeutic effect by 3D-static-spheroid EV treatment - in vivo

### 13.1 Preparation of Moyamoya disease-mimetic animal model and confirmation of changes in cerebral blood flow

A Moyamoya therapeutic effect by 3D-static-spheroid EV treatment was confirmed through a Moyamoya disease-mimetic animal model.

The Moyamoya-mimetic model was fabricated with reference to a method of Roberts JM et al. (Internal carotid artery stenosis: A novel surgical model for moyamoya syndrome. PLoS ONE 13(1): e0191312). Vasoconstriction of the distal internal carotid artery (ICA) and anterior cerebral artery (ACA) in the Circle of Willis in the animal model may be induced using a cerebral carotid artery stenosis method using microcoils. It is also known that the number of anastomoses between the middle cerebral artery and the anterior cerebral artery in the cortical area is significantly reduced to effectively mimick Moyamoya disease.

Surgery and monitoring were performed according to the guidelines of the Laboratory Animal Research Center (LARC; AAALAC International Accredited Facility) of Samsung Medical Center, Seoul. Adult male C57BL/6 mice (10 to 12 weeks old) weighing 20 to 28 g were randomly assigned to sham-operated (n = 3), vehicle experimental group (PBS treatment group, n = 7), and 3D-static-spheroid EV treatment group (n = 8). The mice were provided with a 12-h light/dark cycle in a temperature-controlled facility and freely took food and water.

Bilateral common carotid artery stenosis (BCAS) was induced in C57BL6 mice using a 0.18 mm-diameter coil to fabricate an animal model mimicking Moyamoya disease. Cerebral hypoperfusion could be induced by using a 0.18 mm coil over 3 months without affecting blood pressure. More specifically, the experiment was performed as follows. Anesthesia was induced using 2% isoflurane and maintained under conditions of 1.5% isoflurane in 80% nitrous oxide and 20% oxygen. The rectal temperature was maintained between 36.5°C and 37.5°C. The bilateral common carotid arteries were exposed through a midline cervical incision, and microcoils (Sawane Spring, Japan) with an inner diameter of 0.18 mm were applied to the bilateral common carotid arteries. All animals were randomly assigned to a vehicle experimental group or a 3D-static-spheroid EV treatment group (hereinafter referred to as an EV-treatment group). The EV treatment group received 100 µl of DPBS including 6 × 10⁸ 3D-static-spheroid EVs intravenously (i.v.) via the tail vein daily for 5 days after BCAS surgery. The vehicle experimental group received 100 µl DPBS intravenously via the tail vein daily for 5 days after BCAS surgery in the same manner as the EV treatment group. The mice were sacrificed under anesthesia on day 28 after BCAS.

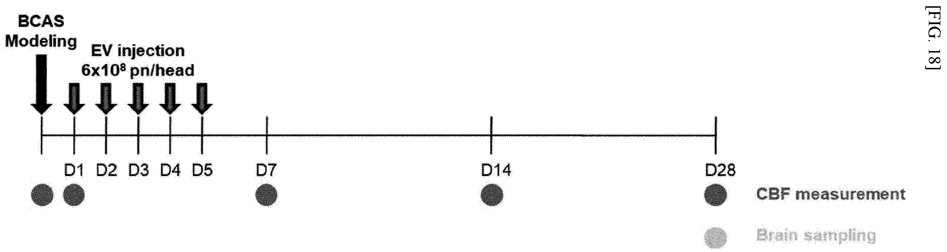

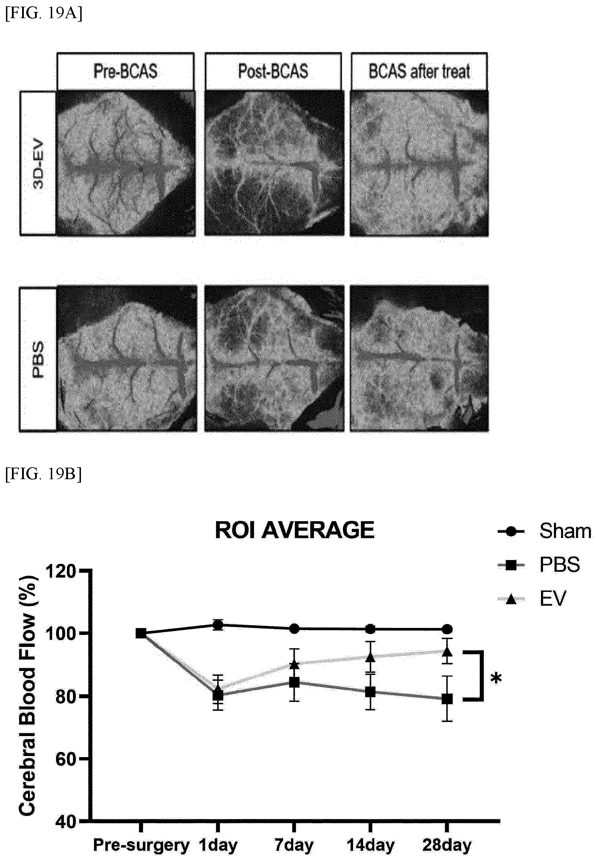

Cerebral blood flow was measured using laser speckle imaging equipment on the next day after fabricating a Moyamoya disease-mimetic animal model. The cerebral blood flow was measured before BCAS surgery, and 1 day, 1 week, 2 weeks, and 4 weeks after surgery. The cerebral blood flow values were expressed as a percentage of the baseline value, and for quantitative analysis, regions of interest (ROIs) were analyzed in at least four areas in each mouse. The experimental protocol was briefly illustrated in FIG. 18, and the cerebral blood flow measurement images and cerebral blood flow quantitative results were illustrated in FIG. 19.

As illustrated in FIGS. 19A and 19B, a significant decrease in cerebral blood flow was confirmed after BCAS surgery. Thereafter, a group administered with PBS showed no recovery in cerebral blood flow, but the 3D-static-spheroid EV treatment group showed a statistically significant increase in cerebral blood flow.

### 13.2 Confirmation of cerebral vascular anastomosis effect by 3D-static-spheroid EV treatment

Moyamoya disease has been currently treated only through surgical methods such as direct and indirect vascular anastomoses. As such, vascular anastomoses are known to be very important in the treatment of Moyamoya disease.

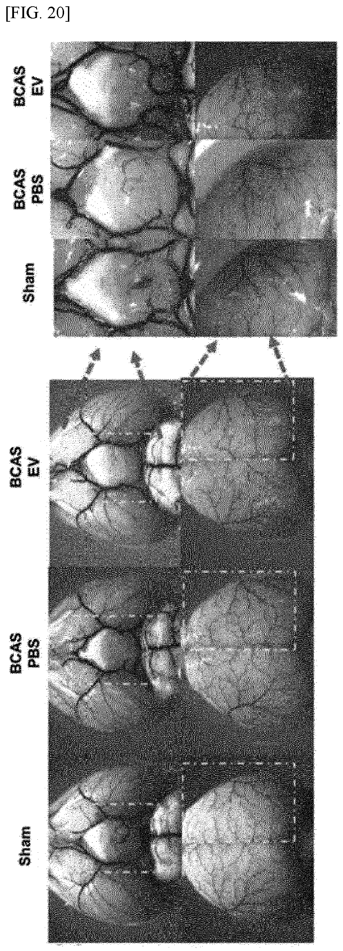

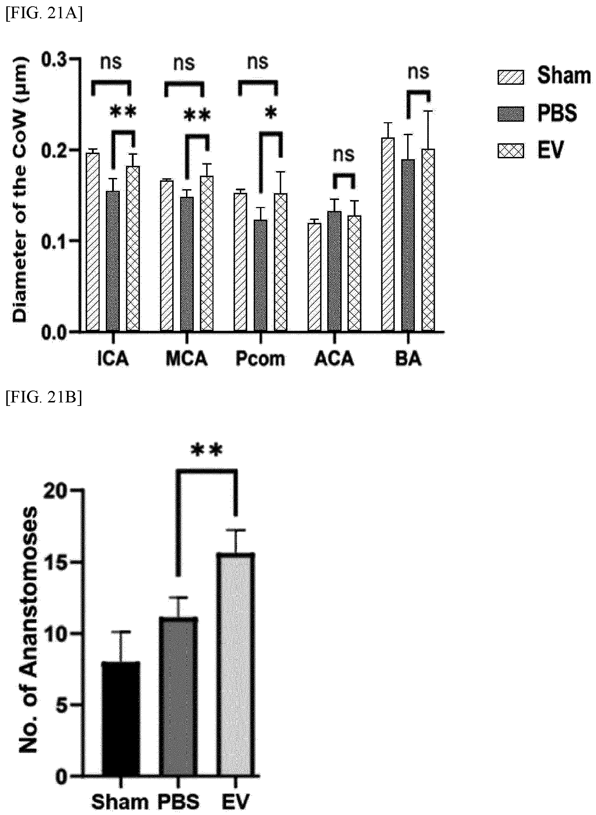

To confirm whether the 3D-static-spheroid EV of the present disclosure may exhibit a vascular anastomosis effect in a Moyamoya disease-mimetic model, an experiment was performed by staining cerebral blood vessels with Indian ink through cardiac perfusion on day 28 of model fabrication and measuring changes in the number of vascular anastomoses. Before staining, a black India ink stock solution (Winsor & Newton, London, UK) was filtered through an RC membrane filter (pore size: 0.2 µm). Mice were sacrificed on the 28th day after BCAS for Indian ink staining. The mice were anesthetized with a mixture of ketamine (60 mg/kg, Yuhan-Yanghaeng, Korea) and xylazine (6 mg/kg, Rompun Inj., Bayer, Berlin, Germany). An aortic catheter (inner diameter 0.76 mm, Vasofix Braunuele, Braun, Melsungen, Germany) was inserted under deep anesthesia, and a hypolethal dose of 50 mg/kg papaverine hydrochloride was injected. After 60 seconds, the mice were perfused with 10 ml of DPBS, and then perfused by 10% Indian ink and 3% gelatin in PBS under pressure-controlled fluid delivery at a temperature of 37°C and 85 mmHg using a peristaltic pump (Miniplus 3, Gilson). The brain was extracted and fixed in 4% paraformaldehyde (PFA) in PBS at 4°C for 48 hours, and then the extracted brain was placed in PBS dissolved with 30% sucrose and stored at 4°C until the brain completely sank to the bottom. Coronal sections (40 µm) were collected from + 0.5 mm to - 0.5 mm around the bregma using a cryostat (cryotom FSE, Thermo Scientific). Thereafter, the sections were mounted on gelatin-coated slides (Marienfeld GmbH, Lauda-Koenigshofen, Germany), dehydrated at room temperature for 30 minutes, and located with a coverslip using a mounting medium (H-5000, Vector Lab, Burlingame, CA, USA). In the sections, the diameters of the internal carotid artery (ICA), the middle cerebral artery (MCA), the posterior communicating artery (Pcom), the anterior cerebral artery (ACA), and the basilar artery (BA) of the Circle of Willis (CoW), and the number of vascular anastomoses of the MCA and the ACA were measured, and the diameter of the Circle of Willis was confirmed. The results of vascular staining were illustrated in FIG. 20, and the results of measuring the changes in the diameter of the Circle of Willis and the number of vascular anastomoses were illustrated in FIG. 21.

As illustrated in FIGS. 21A and 21B, in a vehicle experimental group administered with only PBS after BCAS surgery, a decrease in blood vessel diameter due to stenosis occlusion of the internal carotid artery, which was a characteristic phenomenon of Moyamoya disease, was clearly confirmed in the internal carotid artery, middle cerebral artery, and posterior communicating artery, whereas in the EV treatment group, not only the diameter of the circle of Willis was significantly restored, but also the number of vascular anastomoses was significantly increased. This result shows that the extracellular vesicles of the present disclosure may effectively improve the stenosis occlusion of the internal carotid artery, a decrease in the diameter of the major cerebral blood vessels, a decrease in the collateral circulation blood vessels between the major cerebral blood vessels, and a decrease in cerebral perfusion resulting therefrom, which are representative symptoms of Moyamoya disease or syndrome.

Through the results, it was confirmed that the 3D-static-spheroid EVs of the present disclosure may achieve the vascular anastomosis effect without vascular anastomosis surgery, and restore the diameter of the cerebral blood vessels to restore the cerebral blood flow to a normal level and increase the blood flow in a new direction. This is a result of showing that the EVs of the present disclosure may exhibit a direct Moyamoya therapeutic effect.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

## Claims

1. A pharmaceutical composition for use in preventing or treating Moyamoya disease or Moyamoya syndrome, comprising extracellular vesicles including at least one miRNA selected from the group consisting of miR-19b-3p, miR-99a-5p, miR-100-5p, miR-101-3p and miR-122-5p.

2. The pharmaceutical composition for preventing or treating for use in Moyamoya disease or Moyamoya syndrome of claim 1, wherein the extracellular vesicles highly express the at least one miRNA compared to naturally secreted extracellular vesicles or 2D cultured stem cell-derived extracellular vesicles.

3. The pharmaceutical composition for use in preventing or treating Moyamoya disease or Moyamoya syndrome of claim 2, wherein the extracellular vesicles are a three-dimensional spheroid-type cell aggregate-derived extracellular vesicles prepared by a method comprising the following steps:
(a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.

4. The pharmaceutical composition for use in preventing or treating Moyamoya disease or Moyamoya syndrome of claim 1, wherein the extracellular vesicles induce an increase in at least one miRNA selected from the group consisting of miR-19b-3p, miR-99a-5p, miR-100-5p, miR-101-3p, and miR-122-5p in patients with Moyamoya disease or Moyamoya syndrome.

5. The pharmaceutical composition for use in preventing or treating Moyamoya disease or Moyamoya syndrome of claim 1, wherein the extracellular vesicles are used for improving decreased angiogenic ability and decreased cell proliferation ability of vascular endothelial cells caused by mutation or deletion of a Ring finger protein 213 (RNF213) gene.

6. The pharmaceutical composition for use in preventing or treating Moyamoya disease or Moyamoya syndrome of claim 1, wherein the extracellular vesicles are used for inducing vascular anastomosis, an increase in cerebral blood vessel diameter, or an increase in cerebral blood flow.

7. The pharmaceutical composition for use in preventing or treating Moyamoya disease or Moyamoya syndrome of claim 1, wherein the extracellular vesicles are used for intravenous administration.

8. The pharmaceutical composition for use in preventing or treating Moyamoya disease or Moyamoya syndrome of claim 1, wherein the Moyamoya syndrome secondarily occurs by infection, vasculitis, autoimmune disease, Down syndrome, neurofibromatosis, or radiation therapy for brain tumor.

9. The pharmaceutical composition for use in preventing or treating Moyamoya disease or Moyamoya syndrome of claim 1, wherein the composition is administered before or after direct vascular anastomosis or indirect vascular anastomosis.

10. A pharmaceutical composition for use in preventing or treating Moyamoya disease or Moyamoya syndrome comprising:
extracellular vesicles derived from a three-dimensional spheroid-type cell aggregate prepared by (a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.

11. The pharmaceutical composition for use in preventing or treating Moyamoya disease or Moyamoya syndrome of claim 10, wherein the stem cells are one or more selected from the group consisting of mesenchymal stem cells, pluripotent stem cells, induced pluripotent stem cells, and embryonic stem cells.

12. The pharmaceutical composition for use in preventing or treating Moyamoya disease or Moyamoya syndrome of claim 10, wherein the extracellular vesicles highly express at least one miRNA selected from the group consisting of miR-19b-3p, miR-99a-5p, miR-100-5p, miR-101-3p and miR-122-5p compared to naturally secreted extracellular vesicles or 2D cultured mesenchymal stem cell-derived extracellular vesicles.

13. A pharmaceutical composition for use in preventing or treating Moyamoya disease or Moyamoya syndrome, comprising at least one miRNA selected from the group consisting of miR-19b-3p, miR-99a-5p, miR-100-5p, miR-101-3p and miR-122-5p.

14. The pharmaceutical composition for use in preventing or treating Moyamoya disease or Moyamoya syndrome of claim 13, wherein the miRNA is a form included in a plasmid, a viral vector, or a non-viral carrier.

15. The pharmaceutical composition for use in preventing or treating Moyamoya disease or Moyamoya syndrome of claim 13, wherein the miRNA is a form included in exosomes, microvesicles or extracellular vesicles.

16. The pharmaceutical composition for use in preventing or treating Moyamoya disease or Moyamoya syndrome of claim 15, wherein the extracellular vesicles are extracellular vesicles derived from a three-dimensional spheroid-type cell aggregate prepared by a method comprising the following steps:
(a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.

17. An *in vitro* composition for use in promoting vascular anastomosis of vascular endothelial cells induced by mutation or deletion of a Ring finger protein 213 (RNF213) gene comprising:
extracellular vesicles derived from a three-dimensional spheroid-type cell aggregate prepared by (a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.

18. A method for preparing a composition for preventing or treating Moyamoya disease or Moyamoya syndrome including extracellular vesicles derived from a three-dimensional spheroid-type cell aggregate comprising:
(a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.

19. A method for preventing or treating Moyamoya disease or Moyamoya syndrome comprising: administering to a subject in need thereof extracellular vesicles including at least one miRNA selected from the group consisting of miR-19b-3p, miR-99a-5p, miR-100-5p, miR-101-3p and miR-122-5p.

20. A method for preventing or treating Moyamoya disease or Moyamoya syndrome comprising:
administering to a subject in need thereof extracellular vesicles derived from a three-dimensional spheroid-type cell aggregate prepared by (a) preparing a three-dimensional spheroid-type cell aggregate by 3-dimensionally (3D) static culturing stem cells in a microwell with a diameter of 200 to 800 µm and a depth of 100 to 1,000 µm; and
(b) isolating extracellular vesicles from the three-dimensional spheroid-type cell aggregate.
